# EUROPÄISCHE PATENTANMELDUNG

(11) **EP 0 603 630 A1**
(43) Veröffentlichungstag der Anmeldung: **29.06.1994**
(21) Anmeldenummer: 93119665.3
(22) Anmeldetag: 07.12.1993
(51) Int. Cl.: C07C 29/17, C07C 31/125, C07C 45/50, C07C 45/74, C07C 47/02, C07C 47/21, C07C 69/80, C08K 5/12

(54) **Gemische isomerer Nonanole und Decanole, ihre Herstellung, aus ihnen erhältliche Phthalsäureester und deren Verwendung als Weichmacher**

(30) Priorität: 22.12.1992 DE 4243524
(71) Anmelder: HOECHST AKTIENGESELLSCHAFT, D-65926 Frankfurt am Main (DE)
(72) Erfinder: Bahrmann, Helmut, Dr. Dipl.-Chem., D-46499 Hamminkeln (DE); Fenske, Wilfried, D-46499 Hamminkeln (DE); Greb, Wolfgang, Dr. Dipl.-Chem., D-46535 Dinslaken (DE); Heymanns, Peter, Dr. Dipl.-Chem., D-45147 Essen (DE); Lappe, Peter, Dr. Dipl.-Chem., D-46539 Dinslaken (DE); Müller, Thomas, Dipl.-Ing., D-46535 Dinslaken (DE); Szameitat, Jürgen, Dr. Dipl.-Chem., D-46487 Wesel (DE); Wiebus, Ernst, D-46147 Oberhausen (DE)

(57) **Zusammenfassung**

Gemische isomerer Nonanole und Decanole werden durch gemeinsame Aldolkondensation von n-Butanal und Pentanalen (das sind Gemische aus 60 bis 90 Gew.-% n-Pentanal, 10 bis 40 Gew.-% 2-Methylbutanal und bis zu 1 Gew.-% 3-Methylbutanal), Hydrierung des Aldolkondensationsproduktes zu den gesättigten Alkoholen und Abtrennung der niedriger als die Nonanole und Decanole siedenden Komponenten aus dem Reaktionsgemisch erhalten. Das Alkoholgemisch eignet sich ausgezeichnet zur Herstellung von Esterweichmachern.

## Beschreibung

Die Erfindung betrifft Gemische isomerer Nonanole und Decanole, ein Verfahren zu ihrer Herstellung, die aus diesen Alkoholmischungen erhaltenen Phthalsäureester und deren Verwendung als Weichmacher.

Ester der Phthalsäure finden in großem Umfang als Weichmacher, insbesondere für Polyvinylchlorid, Verwendung. Als Alkoholkomponente werden vorwiegend primäre Alkohole mit 8 bis 10 Kohlenstoffatomen eingesetzt, größte Bedeutung unter ihnen hat gegenwärtig das 2-Ethylhexanol. Phthalsäureester kurzkettigerer Alkohole führen zu Weichmachern mit guter Gelierkraft. Nachteilig ist jedoch ihre höhere Flüchtigkeit. Längerkettige Ester gelieren dagegen langsamer und haben eine schlechtere Kältebeständigkeit.

Die Eigenschaften der Phthalsäureester-Weichmacher werden außer durch die Größe des Alkoholmoleküls auch durch die Verzweigung der Kohlenstoffkette beeinflußt. So ergeben wenig verzweigte Alkohole Esterweichmacher hoher Kälteflexibilität. Weitgehend lineare Alkohole mit 9 bis 10 Kohlenstoffatomen im Molekül gewinnen daher als Alkoholkomponente zunehmende Bedeutung. Voraussetzung für ihren Einsatz ist, daß sie in großen Mengen und kostengünstig zur Verfügung stehen.

Nach der deutschen Patentschrift 2 855 421 werden als Weichmacher Phthalate von C₉-Alkoholen eingesetzt, die durch Oxoreaktion von C₈-Olefinen, Hydrierung des Reaktionsproduktes und Veresterung der C₉-Alkohole mit Phthalsäureanhydrid erhalten werden. 3 bis 20 Gew.-% der Ausgangsolefine sollen in jeder Molekülkette ein Isobutanskelett, weniger als 3 Gew.-% der Olefine quaternären Kohlenstoff aufweisen und mehr als 90 Gew.-% der Gesamtmenge der Olefine als n-Octene, Monomethylheptene und Dimethylhexene vorliegen. Ferner soll das Gewichtsverhältnis der Gesamtmenge der n-Octene und Monomethylheptene zu den Dimethylhexenen mehr als 0,8 betragen.

Phthalsäureester auf Basis von C₁₀-Alkoholen sind Gegenstand der europäischen Patentanmeldung 03 66 089. Die C₁₀-Alkohole werden in Form eines Gemisches eingesetzt, das man durch Hydroformylierung einer Butenfraktion, Aldolkondensation des erhaltenen Aldehydgemisches und anschließende Hydrierung gewinnt. Der Hydroformylierungsschritt unterliegt nach der Verfahrensbeschreibung keinen Beschränkungen. Als Katalysatoren können sowohl Kobalt als auch Rhodium eingesetzt werden, der Zusatz einer organischen Verbindung des dreiwertigen Phosphors wird nicht ausgeschlossen.

Ein anderer Weg zur Gewinnung von Didecylphthalatgemischen ist in der europäischen Patentanmeldung 04 24 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

Nach der Lehre der EP-B-0 052 999 stellt man Weichmacher-Alkohole aus einem Gemisch von Propylen und Butenen im molaren Verhältnis von 2 : 1 bis 1 : 3 her. Die Olefine werden gemeinsam durch Oxoreaktion in ein Gemisch von Butyl- und Amylaldehyden überführt, das einer Aldolkondensationsreaktion unterworfen wird. Die resultierenden Kondensationsprodukte werden anschließend zu gesättigten Alkoholen hydriert.

Die bekannten, zur Herstellung von Weichmachern verwendeten Alkohole bzw. Alkoholgemische erfüllen in wirtschaftlicher und technischer Hinsicht noch nicht alle Anforderungen, die an im industriellen Maßstab erzeugte Produkte gestellt werden, sei es, daß die Ausgangsstoffe nicht in ausreichender Menge und/oder nicht kostengünstig zur Verfügung stehen, daß die Umwandlung der Ausgangsstoffe in die Alkohole an zu aufwendige Prozesse gebunden ist, oder daß die Qualität der aus den Alkoholen hergestellten Weichmacher Wünsche offen läßt.

Es bestand daher die Aufgabe, zur Herstellung von Weichmachern hoher Qualität geeignete Alkohole bzw. Alkoholgemische zu entwickeln. Sie sollen aus preiswert zur Verfügung stehenden Rohstoffen in technisch einfacher Weise erhalten werden.

Diese Aufgabe wird gelöst durch Gemische isomerer Nonanole und Decanole, die erhalten wurden durch gemeinsame Aldolkondensation von n-Butanal und Pentanalen im Molverhältnis 1 : 2 bis 1 : 10, wobei die Pentanale Gemische aus 60 bis 90 Gew.-% n-Pentanal, 10 bis 40 Gew.-% 2-Methylbutanal und bis zu 1 Gew.-% 3-Methylbutanal sind, Hydrierung des Aldolkondensationsproduktes zu den gesättigten Alkoholen und Abtrennung der niedriger als die Nonanole und Decanole siedenden Komponenten aus dem Reaktionsgemisch.

Bevorzugt sind Gemische isomerer Nonanole und Decanole, die aus n-Butanal und Pentanalen hergestellt werden, die 65 bis 80 Gew.-% n-Pentanal, 20 bis 35 Gew.-% 2-Methylbutanal und bis zu 1 Gew.-% 3-Methylbutanal enthalten.

Die Alkoholgemische werden durch Aldolkondensation von n-Butanal und Pentanalen im Molverhältnis von 1 zu 2 bis 1 zu 10 enthaltender Gemische, anschließende Hydrierung des Aldolkondensationsproduktes und Abtrennung entstandenen 2-Ethylhexanols gewonnen. Die Herkunft der Aldehyde ist beliebig, sie richtet sich insbesondere nach den wirtschaftlichen Gegebenheiten. Um die Bildung nur wenig verzweigter Alkohole zu fördern, müssen sie jedoch die Bedingung erfüllen, die Carbonylgruppe am endständigen Kohlenstoffatom zu tragen und, im Falle der Pentanale, zumindest weitgehend unverzweigt zu sein. Daher werden als Pentanale Gemische eingesetzt, die 60 bis 90 Gew.-% n-Pentanal, 10 bis 40 Gew.-% 2-Methylbutanal und bis zu 1 Gew.-% 3-Methylbutanal enthalten.

Bevorzugt werden durch Hydroformylierung (Oxosynthese) von Propylen bzw. Butenen hergestellte Aldehyde als Ausgangsmaterial verwendet. Die erforderlichen Olefine stehen in technischen Mengen zur Verfügung. Propylen fällt als Nebenprodukt bei der Ethylenerzeugung durch Pyrolyse von Kohlenwasserstoffgemischen in Gegenwart von Wasserdampf sowie bei einigen Raffinerie-Prozessen, insbesondere dem katalytischen Cracken von Erdölfraktionen, an.

Auch Buten-1 und Buten-2 enthaltende Gemische werden als Raffinerienebenprodukte bei der Herstellung von Automobiltreibstoffen und bei der Herstellung von Ethylen durch thermische Spaltung höherer Kohlenwasserstoffe zwangsweise in erheblichen Mengen erhalten. Man gewinnt sie aus den C₄-Crackschnitten des Pyrolyseproduktes durch Extraktion des Butadiens-1,3 mit einem selektiven Lösungsmittel und anschließende Abtrennung des Isobutens vorzugsweise durch Umwandlung in Methyl-tert.-butylether. Statt das Butadien-1,3 zu extrahieren, kann es auch im C₄-Crackschnitt partiell zu Butenen hydriert werden. Das von Butadien-1,3 befreite Pyrolyseprodukt wird als Raffinat I bezeichnet. Ist darüber hinaus auch noch das Isobuten abgetrennt, spricht man von Raffinat II. Dieses Buten-1/Buten-2-Gemisch ist besonders geeignet zur Weiterverarbeitung zu C₁₀-Alkoholen.

Grundsätzlich sind alle gängigen, technisch ausgeübten Hydroformylierungsverfahren zur Umwandlung der Olefine in Aldehyde geeignet. Sie kann also in Gegenwart von Kobalt- oder auch Rhodiumkatalysatoren bei Drücken von 10 bis 35 MPa und Temperaturen von 120 bis 180°C, ebenso erfolgen wie in Gegenwart von Kobalt/Phosphin-Katalysatoren bei Drücken von 5 bis 10 MPa oder auch in Gegenwart von Rhodiumkatalysatoren, die durch Phosphin modifiziert sind, bei Temperaturen von 60 bis 150°C und Drücken von 1 bis 8 MPa. Bei der zuletzt beschriebenen Variante der Hydroformylierungsreaktion kann der Katalysator homogen im Reaktionsgemisch gelöst vorliegen oder gegenüber dem Reaktionsgemisch eine eigene Phase bilden.

Zur Herstellung der Aldehyde setzt man Propylen und die Butene gemeinsam, vorzugsweise jedoch getrennt um. Besonders bewährt hat es sich, die Hydroformylierung als heterogene Reaktion in einem Zweiphasensystem durchzuführen, eine Reaktion, die z.B. in der DE-C-26 27 354 beschrieben ist. Diese Ausführungsform der Oxosynthese stellt sicher, daß aus Olefinen, deren Doppelbindung sich an einem endständigen Kohlenstoffatom befindet, in hohem Maße n-Aldehyde gebildet werden und eine Isomerisierung der Olefine durch Wanderung der Doppelbindung während der Umsetzung weitgehend unterbleibt.

Der Zweiphasenprozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist bekannt und z.B. in DE-PS 26 27 354 und DD-PS 259 194 beschrieben. Die Reaktion der Olefine erfolgt bei Temperaturen von 70 bis 150°C, vorzugsweise 100 bis 130°C und unter Drücken im Bereich von 0,4 bis 30, insbesondere 1 bis 10 MPa mit Wassergas, das Kohlenmonoxid und Wasserstoff im Volumenverhältnis 1 : 10 bis 10 : 1 enthält. Die Rhodiumkonzentration beträgt 20 bis 1000 Gew.-ppm, vorzugsweise 50 bis 500 Gew.-ppm, bezogen auf die wäßrige Katalysatorlösung, je mol Rhodium setzt man 4 bis 100 mol wasserlösliches Phosphin ein. Das Volumverhältnis von wäßriger zu organischer Phase beträgt 0,1 bis 10 : 1.

Der Umsatz der Butene wird deutlich erhöht, wenn man der wäßrigen Katalysatorlösung ein Phasentransferreagenz (Lösungsvermittler) zusetzt. Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 4 Kohlenstoffatomen bedeuten und E insbesondere für Sulfat, Tetrafluoroborat, Acetat, Methosulfat, Benzolsulfonat, Alkylbenzolsulfonat, Toluolsulfonat, Lactat oder Citrat steht.

Propylen wird nach dem beschriebenen Verfahren bis zu 99% umgesetzt, das erhaltene Butanalgemisch besteht zu über 95 Gew.-% aus der n-Verbindung. Bei Einsatz von Buten-1-/Buten-2-Gemischen reagiert vorwiegend Buten-1. Je nach den gewählten Reaktionsparametern werden Buten-1 bzw. Buten-2 zu mehr als 95 % umgesetzt. Es bilden sich 60 bis 90 Gewichtsprozent n-Pentanal, der Rest besteht aus 2-Methylbutanal und gegebenenfalls 3-Methylbutanal.

Nach Beendigung der getrennt oder gemeinsam durchgeführten Hydroformylierung werden die Aldehyde vom Katalysator, von den nichtumgesetzten Reaktionsteilnehmern und den übrigen Reaktionsprodukten abgetrennt. Das geschieht im Falle der heterogenen Reaktion durch einfache Phasentrennung. Bei Umsetzung in homogener Phase ist die Destillation das übliche Trennverfahren.

In der sich anschließenden Aldolkondensation werden Gemische eingesetzt, die je mol n-Butanal 2 bis 10 mol, insbesondere 7 bis 10 mol Pentanale enthalten. Die Umsetzung des Aldehydgemischs erfolgt auf konventionellem Wege unter der Einwirkung basischer Katalysatoren. Eine Vorbehandlung der Aldehyde, z.B. eine spezielle Reinigung, ist nicht erforderlich. Jedoch empfiehlt es sich, im Falle der Butanale aus dem C₄-Aldehydgemisch i-Butanal destillativ zu entfernen, sofern sein Anteil im Gemisch etwa 2 Gew.-% übersteigt. Als Katalysatoren finden Alkalicarbonate oder Alkalihydroxide, insbesondere Verbindungen des Natriums oder Kaliums und Amine, vorzugsweise tertiäre Amine, wie Triethylamin, Tri-n-propylamin, Tri-n-butylamin, Anwendung. Man arbeitet bei Temperaturen von 60 bis 160°C, insbesondere 80 bis 130°C und bei Normaldruck oder bei bis etwa 1 MPa erhöhtem Druck. Die Reaktionszeit beträgt wenige Minuten bis zu mehreren Stunden und ist insbesondere abhängig von Katalysatortyp und Reaktionstemperatur. Aufgrund ihrer höheren Reaktivität reagieren vornehmlich die geradkettigen Aldehyde. Durch Selbstkondensation von n-Butanal bzw. n-Pentanal entstehen C₈- bzw. C₁₀-enale und die Mischkondensation von n-Butanal und n-Pentanal ergibt C₉-enale. Die Reaktionen zwischen n-Butanal bzw. n-Pentanal und verzweigtkettigen Pentanalen verlaufen mit deutlich verminderter Geschwindigkeit, die Umsetzung verzweigtkettiger Pentanale untereinander tritt weitgehend in den Hintergrund.

Das durch Kondensation erhaltene Gemisch ungesättigter Aldehyde wird anschließend zu einem Gemisch hydriert, das Nonyl- und Decylalkohole, daneben noch 2-Ethylhexanol und aus gegebenenfalls nicht durch Aldolkondensation umgesetzten C₄- und C₅-Aldehyden Butanole und Pentanole enthält. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Aus dem anfallenden Alkoholgemisch werden erfindungsgemäß 2-Ethylhexanol und weitere Alkohole und auch Verunreinigungen, die niedriger als die Nonanole und Decanole sieden, durch Destillation bei 100 bis 125°C und 1 bis 4 kPa (10 bis 40 mbar) Druck abgetrennt.

Das verbleibende Gemisch aus Nonanolen und Decanolen eignet sich vorzüglich als Alkoholkomponente in Phthalsäureestern, die als Weichmacher verwendet werden sollen. Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem C₉-, C₁₀-Alkoholgemisch im Molverhältnis 1 : 2 bis 1 : 3 in einer Stufe um. Die Reaktionsgeschwindigkeit kann durch Katalysatoren und/oder durch Erhöhung der Reaktionstemperatur gesteigert werden. Um das Gleichgewicht in Richtung der Esterbildung zu verschieben, ist es erforderlich, das gebildete Wasser aus dem Reaktionsgemisch zu entfernen.

Die aus dem erfindungsgemäßen Nonanol-/Decanolgemisch erhaltenen Phthalate zeichnen sich durch geringe Flüchtigkeit und gute Gelierfähigkeit aus.

### Beispiel 1

Zu 980,0 g 2,5 %iger NaOH (0,61 mol), die unter Stickstoffschutz auf 60°C erwärmt wurden, gibt man über einen Zeitraum von 20 min eine Mischung aus 272,8 g (3,17 mol) n-Valeraldehyd, 181,8 g (2,11 mol) 2-Methylbutanal und 45,5 g (0,63 mol) n-Butyraldehyd. Das Gemisch wird eine Stunde unter Rückfluß auf 88 bis 90°C erhitzt. Nach Abkühlung auf 30°C trennen sich organische und wäßrige Phase voneinander.

Das Aldolkondensationsprodukt wird in Gegenwart eines Nickelkatalysators bei 10 MPa Druck und 140°C hydriert. Nach Abfiltrieren des Katalysators erhält man ein Rohalkoholgemisch folgender gaschromatographisch ermittelter Zusammensetzung (Gew.-%):

| | |
|---|---|
| Vorlauf | 0,2 |
| 2-Methylbutanol | 30,2 |
| n-Pentanol | 0,3 |
| 2-Ethylhexanol | 1,6 |
| 2-Ethyl-4-methylhexanol | 2,1 |
| 2-Propylhexanol | 6,4 |
| 2-Ethylheptanol | 6,2 |
| 2-Propyl-4-methylhexanol | 14,7 |
| 2-Propylheptanol | 35,4 |
| Nachlauf | 2,9 |

Die destillative Abtrennung der Pentanole und des 2-Ethylhexanols liefert ein Alkoholgemisch folgender Zusammenstzung (Gew.-%)

| | |
|---|---|
| 2-Ethyl-4-methylhexanol | 3,2 |
| 2-Propylhexanol | 9,9 |
| 2-Ethylheptanol | 9,6 |
| 2-Propyl-4-methylhexanol | 22,6 |
| 2-Propylheptanol | 54,7 |

Die Veresterung mit Phthalsäureanhydrid geschieht in Gegenwart von Schwefelsäure als Katalysator und von Cyclohexan zur azeotropen Entfernung des Reaktionswassers. Nach Neutralisation, Alkoholabtrennung und Trocknung erhält man ein Phthalsäureestergemisch isomerer Nonanole und Decanole, das bei 20°C eine Dichte von 0,967 g/ml und eine Viskosität von 138 mPa.s zeigt.

### Beispiel 2

Zu 980,0 g 2,5 %iger NaOH (0,61 mol), die unter Stickstoffschutz auf 60°C erwärmt wurden, gibt man über einen Zeitraum von 20 min tropfenweise eine Mischung aus 214,3 g (2,49 mol) n-Valeraldehyd, 143,0 g (1,66 mol) 2-Methylbutanal und 142,8 g (1,98 mol) n-Butyraldehyd. Das Gemisch wird eine Stunde unter Rückfluß auf 89 bis 92°C erhitzt. Nach Abkühlen auf 30°C trennen sich organische und wäßrige Phase voneinander.

Die Hydrierung des Kondensationsproduktes analog Beispiel 1 ergibt ein Rohalkoholgemisch folgender gaschromatographisch ermittelter Zusammensetzung (Gew.-%):

| | |
|---|---|
| Vorlauf | 0,2 |
| 2-Methylbutanol | 20,9 |
| n-Pentanol | 0,2 |
| 2-Ethylhexanol | 10,9 |
| 2-Ethyl-4-methylhexanol | 5,2 |
| 2-Propylhexanol | 13,9 |
| 2-Ethylheptanol | 13,5 |
| 2-Propyl-4-methylhexanol | 10,0 |
| 2-Propylheptanol | 22,3 |
| Nachlauf | 2,9 |

Die destillative Abtrennung der Pentanole und des 2-Ethylhexanols liefert ein Alkoholgemisch folgender Zusammensetzung (Gew.-%):

| | |
|---|---|
| 2-Ethyl-4-methylhexanol | 8,0 |
| 2-Propylhexanol | 21,4 |
| 2-Ethylheptanol | 20,8 |
| 2-Propyl-4-methylhexanol | 15,4 |
| 2-Propylheptanol | 34,4 |

Nach der Veresterung mit Phthalsäureanhydrid analog Beispiel 1 erhält man ein Estergemisch, das eine Viskosität von 118 mPa.s und eine Dichte von 0,969 g/ml aufweist.

### Beispiel 3

Eine Mischung aus 280,0 g (3,25 mol) n-Valeraldehyd, 120,0 g (1,39 mol) 2-Methylbutanal und 120,0 g (1,66 mol) n-Butyraldehyd werden gemäß Beispiel 1 in Gegenwart von 1016 g 2,5 %iger NaOH (0,63 mol) aldolisiert.

Die Hydrierung des Kondensationsproduktes analog Beispiel 1 ergibt ein Rohalkoholgemisch folgender Zusammensetzung (Gew.-%):

| | |
|---|---|
| Vorlauf | 0,7 |
| 2-Methylbutanol | 17,9 |
| n-Pentanol | 0,3 |
| 2-Ethylhexanol | 7,0 |
| 2-Ethyl-4-methylhexanol | 3,6 |
| 2-Propylhexanol | 13,8 |
| 2-Ethylheptanol | 13,5 |
| 2-Propyl-4-methylhexanol | 9,5 |
| 2-Propylheptanol | 31,7 |
| Nachlauf | 2,0 |

Nach destillativer Aufarbeitung erhält man ein Alkoholgemisch folgender Zusammensetzung (Gew.-%):

| | |
|---|---|
| 2-Ethyl-4-methylhexanol | 5,1 |
| 2-Propylhexanol | 19,2 |
| 2-Ethylheptanol | 18,7 |
| 2-Propyl-4-methylhexanol | 13,1 |
| 2-Propylheptanol | 43,9 |

Die Veresterung mit Phthalsäureanhydrid gemäß Beispiel 1 führt zu einem Estergemisch mit einer Viskosität von 123 mPa.s und einer Dichte von 0,969 g/ml.

### Beispiele 4 bis 7

Die Beispiele 4 bis 7 werden entsprechend Beispiel 1 durchgeführt, Angaben zur Zusammensetzung des Einsatzgemisches, des Rohalkoholgemisches und des für die Weichmachergewinnung geeigneten Alkoholgemisches sind der nachfolgenden Tabelle zu entnehmen.

| | Beispiel 4 | Beispiel 5 | Beispiel 6 | Beispiel 7 |
|---|---|---|---|---|
| Einsatzgemisch n-Valeraldehyd | | | | |
| (g) | 156,7 | 208,9 | 139,3 | 234,8 |
| (mol) | 1,82 | 2,43 | 1,62 | 2,73 |

| 2-Methylbutanal | | | | |
|---|---|---|---|---|
| (g) | 17,4 | 52,0 | 34,8 | 26,1 |
| (mol) | 0,20 | 0,60 | 0,40 | 0,30 |

| n-Butyraldehyd | | | | |
|---|---|---|---|---|
| (g) | 74,3 | 22,3 | 74,3 | 22,3 |
| (mol) | 1,03 | 0,31 | 1,03 | 0,31 |

| 2,5 %ige NaOH | | | | |
|---|---|---|---|---|
| (g) | 480,5 | 528,0 | 480,0 | 528,5 |
| (mol) | 0,30 | 0,33 | 0,30 | 0,33 |

| Rohalkoholgemisch (in Gew.-%): | | | | |
|---|---|---|---|---|
| Vorlauf | 0,2 | 0,2 | 0,2 | 0,2 |
| 2-Methylbutanol | 4,6 | 13,1 | 9,9 | 6,5 |
| n-Pentanol | 0,3 | 0,3 | 0,2 | 0,3 |
| 2-Ethylhexanol | 10,2 | 0,9 | 10,9 | 0,9 |
| 2-Ethyl-4-methylhexanol | 1,4 | 0,9 | 2,8 | 0,5 |
| 2-Propylheptanol | 19,0 | 6,6 | 17,6 | 7,0 |
| 2-Ethylheptanol | 18,5 | 6,4 | 16,9 | 6,7 |
| 2-Propyl-4-methylhexanol | 3,3 | 10,1 | 6,2 | 6,1 |
| 2-Propylheptanol | 40,6 | 59,6 | 33,2 | 69,8 |
| Nachlauf | 1,9 | 1,9 | 2,1 | 2,0 |

| Weichmacheralkoholgemisch (in Gew.-%): | | | | |
|---|---|---|---|---|
| 2-Ethyl-4-methylhexanol | 1,8 | 1,0 | 3,6 | 0,6 |
| 2-Propylhexanol | 22,9 | 7,9 | 23,0 | 7,8 |
| 2-Ethylheptanol | 22,3 | 7,6 | 22,0 | 7,4 |
| 2-Propyl-4-methylhexanol | 4,0 | 12,1 | 8,2 | 6,8 |
| 2-Propylheptanol | 49,0 | 71,4 | 43,2 | 77,4 |

Die ausgezeichnete Gelierfähigkeit der aus dem erfindungsgemäßen Alkoholgemisch hergestellten Phthalsäureester-Weichmacher zeigt sich bei einem Vergleich mit den als Weichmacher eingeführten Di(isodecyl)phthalaten (DIDP).

Zur Beurteilung der Gelierfähigkeit wurde die kritische Lösetemperatur nach DIN 53 408 bestimmt

| | Phthalsäureester unter Verwendung von Alkoholgemischen nach | | | DIDP |
|---|---|---|---|---|
| | Beispiel 1 | Beispiel 2 | Beispiel 3 | |
| kritische Lösetemperatur (°C) | 139 | 133 | 135 | 140 |

Die hervorragende Gelierfähigkeit der aus den neuen Alkoholgemischen erhaltenen Phthalsäureester folgt aus der kritischen Lösetemperatur, die niedriger liegt als bei DIDP.

## Patentansprüche

1. Gemische isomerer Nonanole und Decanole erhalten durch gemeinsame Aldolkondensation von n-Butanal und Pentanalen im Molverhältnis 1 : 2 bis 1 : 10, wobei die Pentanale Gemische aus 60 bis 90 Gew.-% n-Pentanal, 10 bis 40 Gew.-% 2-Methylbutanal und bis zu 1 Gew.-% 3-Methylbutanal sind, Hydrierung des Aldolkondensationsproduktes zu den gesättigten Alkoholen und Abtrennung der niedriger als die Nonanole und Decanole siedenden Komponenten aus dem Reaktionsgemisch.

2. Gemische isomerer Nonanole und Decanole nach Anspruch 1, dadurch gekennzeichnet, daß die Pentanale Gemische aus 65 bis 80 Gew.-% n-Pentanal, 20 bis 35 Gew.-% 2-Methylbutanal und bis zu 1 Gew.-% 3-Methylbutanal sind.

3. Verfahren zur Herstellung von Gemischen isomerer Nonanole und Decanole nach Anspruch 1 oder 2, dadurch gekennzeichnet, daß man Propylen und die Butene getrennt voneinander hydroformyliert, Mischungen der erhaltenen Butanale und Pentanale in Gegenwart basischer Katalysatoren kondensiert, das Kondensationsprodukt zu gesättigten Alkoholen hydriert und aus dem Reaktionsgemisch die niedriger als die Nonanole und Decanole siedenden Komponenten abtrennt.

4. Verfahren nach Anspruch 3, dadurch gekennzeichnet, daß die Olefine in Gegenwart einer wäßrigen Lösung von Rhodium-Phosphin-Komplexverbindungen als Katalysator bei 70 bis 150°C und 0,4 bis 30 MPa Druck hydroformyliert werden.

5. Verfahren nach Anspruch 3 oder 4, dadurch gekennzeichnet, daß man Mischungen aus Butanalen und Pentanalen bei 60 bis 160°C in Gegenwart tertiärer Amine kondensiert.

6. Verfahren nach einem oder mehreren der Ansprüche 3 bis 5, dadurch gekennzeichnet, daß das Kondensationsprodukt in Gegenwart von Nickelkatalysatoren bei 100 bis 180°C und 1 bis 10 MPa Druck hydriert wird.

7. Verfahren nach einem oder mehreren der Ansprüche 3 bis 6, dadurch gekennzeichnet, daß aus dem Hydrierprodukt durch Destillation bei 100 bis 125°C und 1 bis 4 kPa (10 bis 40 mbar) Druck die leichter als die Nonanole und die Decanole siedenden Komponenten des Hydrierproduktes abgetrennt werden.

8. Weichmacher erhalten durch Veresterung von Alkoholgemischen nach Anspruch 1 oder 2 mit Phthalsäure oder Phthalsäureanhydrid.
